# EUROPEAN PATENT APPLICATION

(11) **EP 2 604 316 A2**
(43) Date of publication of application: **19.06.2013**
(21) Application number: 12186231.2
(22) Date of filing: 27.09.2012
(51) Int. Cl.: A61Q 17/04, A61K 8/34, A61K 8/35, A61K 8/37

(54) **Topical sunscreen compositions**

(30) Priority: 28.09.2011 US 201113246962
(71) Applicant: JOHNSON & JOHNSON CONSUMER COMPANIES, INC., Skillman, New Jersey 08558 (US)
(72) Inventor: Daly, Susan, Basking Ridge, NJ New Jersey 07920 (US); Tokgoz-Engrand, Selcan, Princeton, NJ New Jersey 08540 (US)
(74) Representative: Kirsch, Susan Edith

(57) **Abstract**

Substantially anhydrous compositions including at least 10% by weight of a C₂ or C₃ mono-alcohol, a solid, organic UV-filter dissolved therein; and an oil portion including one or more oils, the oil portion having an interfacial tension with water of greater than 9 and less than 32.5, where the composition has a volume fraction of oil relative to oil plus C₂ or C₃ mono-alcohol that is about 0.44 or greater, and is substantially free of a mixture of a low-volatility, C₂ - C₈ liquid silicone and a branched fatty acid ester of a polyprotic carboxylic acid in a ratio of from about 3:1 to 4.25:1

## Description

### FIELD OF THE INVENTION

The present invention relates to topical sunscreen compositions having the ability to be successfully applied to wet skin.

### BACKGROUND OF THE INVENTION

Conventional sunscreen products generally take the form of ultraviolet (UV)-filter compounds and/or particulate UV-screening compounds (collectively, "sunscreen actives") that are solublized, emulsified, or dispersed in a vehicle, which is topically applied to the skin. The sunscreen actives, typically through the aid of polymers and other ingredients included in the vehicle, form a thin, protective, and often water-resistant layer on the skin.

The applicants have recognized that, while certain products are successful at providing a durable protective barrier when applied to dry skin, such is not typically the result when applied to skin that is damp with sweat or has residual water thereon. In fact, when applied to wet skin, the tendency of conventional sunscreen products is to dilute the sunscreen actives, smear, and form an incomplete film, often one that flakes or peels off the skin, and/or takes on a pasty, white appearance. The end result is unattractive, and renders the skin with poor protection from the sun's rays.

Others have contemplated a solution to this problem by using a water-in-oil emulsifier to "self-emulsify," presumably in the presence of residual water present on the skin. However, the applicants have recognized that severe aesthetic and performance problems still exist in most all "wet skin" sunscreen products. Accordingly, the applicants have now identified a novel sunscreen composition that is suitable for use on wet skin and resists the tendency to whiten in the presence of residual water.

### SUMMARY OF THE INVENTION

The present invention relates to substantially anhydrous compositions comprising at least 10% by weight of a C₂ or C₃ mono-alcohol; an organic UV-filter dissolved in the composition; and an oil portion comprising one or more oils. The oil portion is miscible with the C₂ or C₃ mono-alcohol and has an interfacial tension with water of greater than 9 and less than 32.5. The composition has a volume fraction of oil portion relative to the sum of the oil portion plus the C₂ or C₃ mono-alcohol that is greater than 0.44. The composition is substantially free of a mixture of a low-volatility, C₂ - C₈ liquid silicone and a branched fatty acid ester of a polyprotic carboxylic acid in a ratio of from about 3:1 to 4.25:1.

### DETAILED DESCRIPTION OF THE INVENTION

It has been discovered that solid, organic ultraviolet (UV) sunscreen filters can be dissolved in a composition comprising a C₂ or C₃ mono-alcohol, and an oil portion comprising one or more oils, to form a substantially anhydrous composition that surprisingly resists whitening in the presence of water.

As used herein, "cosmetically acceptable" means suitable for use in contact with tissues (e.g., the skin) without undue toxicity, incompatibility, instability, irritation, allergic response, or the like.

As used herein, "substantially free" means the composition contains less than about 1, such as less than about 0.1, e.g., less than about 0.01 weight percent of an ingredient.

Compositions of the present invention are substantially anhydrous. By "substantially anhydrous" it is meant that the composition is substantially free of water.

The term "comprising" encompasses "including" as well as "consisting and "consisting essentially of", e.g. a composition "comprising" X may consist exclusively of X or may include something additional e.g. X and Y.

### Concentration Ranges and "Concentrate"

Unless otherwise specifically described, all percentages included herein are percentages by weight, based on total weight of the composition, excluding any propellant that may be present. For compositions that include a propellant, the term "concentrate" is used to refer to that portion of the composition that excludes propellant.

### Dissolved, Solid, Organic UV-filter

Compositions of the present invention include a dissolved, solid, organic UV filter. Solid, organic UV-filters that are useful in the present invention are cosmetically-acceptable compounds that absorb radiation in the UV range, are solid at ambient temperature (22°C) and pressure (1 atmosphere), and are generally soluble in one or more organic hydrocarbon solvents. The organic, UV filter absorbs radiation in some portion of the ultraviolet spectrum (290nm-400nm), and may have an extinction coefficient of about 1000 mol⁻¹ cm⁻¹ or more, for example greater than 10,000 or 100,000 or 1,000,000 mol⁻¹ cm⁻¹, for at least one wavelength within the above-defined ultraviolet spectrum. The organic UV-filter, which is solid at ambient temperature, is dissolved and homogeneously distributed in the composition (exclusive of any propellant).

Examples of solid organic UV-filters that may be dissolved in compositions of the present invention include, without limitation, benzophenone-3 (i.e., oxybenzone), 2-Hydroxy-4-methoxyphenyl)-(2-hydroxyphenyl)methanone (i.e., dioxybenzone), 2-(2H-Benzotriazol-2-yl)-4-methylphenol, also known as drometrizole trisiloxane (also known as MEXORYL XL), butylmethoxy dibenzoylmethane ("avobenzone"), 4-methyl benzilidene camphor ("4-MBC"), ethylhexyl triazone (available as UVINUL T-150 from BASF of Ludwigshafen, Germany), diethylamino hydroxybenzoyl hexyl benzoate ("DHHB") available as UVINUL A Plus from BASF; and Bemotrizinol (available as TINOSORB S from BASF), Menthyl-2-aminobenzoate ("menthyl anthranilate"), 4-Aminobenzoic acid ("PABA"), hydroxy methylphenyl benzotriazole, and combinations thereof.

The solid, organic UV-filters may be selected from the group consisting of oxybenzone, avobenzone, drometrizole trisiloxane, ethylhexyl triazone, DHHB, and Bemotrizinol. The solid, organic UV-filters may be selected from the group consisting of oxybenzone and avobenzone or may consist of oxybenzone and avobenzone.

The dissolved organic, UV-filter may be present in a concentration range from about 0.5% to about 30%, such as from about 1% to about 20%, such as from about 1.5% to about 15%, such as from about 1.5% to about 10%, such as from about 2% to about 10%, e.g., from about 3% to about 9% by weight. The dissolved organic UV filter may be present in a concentration from about 0.5% to about 9%.

### C₂ or C₃ Mono-alcohol

Compositions of the present invention include a C₂ or C₃ mono-alcohol. By "C₂ or C₃ mono-alcohol" it is meant ethanol, propanol, isopropanol, or combinations thereof. The C₂ or C₃ mono-alcohol is preferably ethanol. The C₂ or C₃ mono-alcohol is present in a concentration of about 10% or more by weight. The C₂ or C₃ mono-alcohol may be present in a concentration from about 10% to about 57%, such as from about 12% to about 56%, such as from about 15% to about 56%, such as from about 20% to about 56%, e.g., from about 25% to about 55% by weight.

### Oil Portion

Compositions of the present invention include an oil portion. By "oil portion," it is meant, collectively, all of the oils present in the composition (i.e., no oils present in the composition are excluded from the oil portion). The oil portion, in aggregate, has a moderate polarity. As such, the oil portion has an interfacial tension with water that is greater than 9 and less than 32.5. The oil portion may have an interfacial tension with water that is from about 10 to about 32.5, such as from about 11.6 to about 32.5, such as from about 11.6 to about 31.9, such as from about 11.6 to about 25, e.g., from about 11.6 to about 20. The oil portion of the composition may have an interfacial tension with water that is equal to or greater than that of diisopropyl adipate and equal to or less than that of a C₁₂-C₁₈ alkyl benzoate (Tegosoft TN, available from Evonik Goldschmidt GmbH of Essen, Germany).

The interfacial tension of the oil portion may be determined by the "Pendant Drop Interfacial Tension Test," as described below. A contact angle meter, such as the (Dynamic) Contact Angle Meter model DM-701, made by Kyowa Interface Science Co., Ltd. (Tokyo, Japan), equipped with FAMAS interface measurement analysis system software, is used. A reservoir on the meter is filled with deionized water, and a glass syringe is filled with test oil. When the oil portion is less dense than the water, an inverted 22-gauge stainless steel needle is attached to the syringe, from which a small amount of the oil is pushed into the water reservoir to form a drop at the end of the needle. When the oil portion is denser than the water, a normal 22-gauge stainless steel needle is used. After the drop is formed at the end of the needle, sufficient time is given for each drop to reach a point of stability. Once the drop is stable, images of the drop are captured with a fast image capturing camera (up to 60 frames per second) and the software calculates the interfacial tension of the oil with water based on the shape of the drop in the captured image. A new liquid droplet is created for each measurement. The distilled water medium is changed and the container is cleaned for every measurement to minimize influence from liquid cross-mixing. The interfacial tension values are measured in triplicate for each oil sample.

In order to standardize the results and allow for some variance in measurement for the Pendant Drop Interfacial Tension Test, diisopropyl adipate is assigned a standard interfacial tension value of 11.6. At the time of each test, diisopropyl adipate is tested to determine the value of diisopropyl adipate tested vs. the standard interfacial tension for diisopropyl adipate. If the value for the tested diisopropyl adipate is determined to be different than 11.6, then the subsequent values for interfacial tension for other oils are adjusted upward or downward for situations where the value measured for diisopropyl adipate is greater or less than 11.6, respectively. The adjusting factor is the ratio of the measured value for diisopropyl adipate divided by 11.6. For example, if diisopropyl adipate is measured at the time as 13.1, then the reported values will be multiplied by 13.1/11.6, i.e. 1.13.

The oil portion includes one or more oils. By "oil," it is meant a hydrophobic compound that has a melting point that is below 30°C and is insoluble in water. Although all of the one or more oils of the oil portion may be selected to have an interfacial tension with water that is greater than 9 and less than 32.5, each individual oil present in the oil portion need not meet this requirement, so long as the oil portion, as a whole, meets the requirement. The one or more oils may be selected to meet one or more of the following three criteria: (a) has a carbon chain of at least four carbons in which none of the four carbons is a carbonyl carbon; (b) has two or more alkyl siloxy groups; or (c) has two or more oxypropylene groups in sequence. The hydrophobic moiety may include linear, cyclic, aromatic, saturated or unsaturated groups. Compounds that are amphiphilic may be excluded from the definition of "oil" and such compounds that have hydrophilic moieties, such as anionic, cationic, zwitterionic, or nonionic groups, that are polar, including sulfate, sulfonate, carboxylate, phosphate, phosphonates, ammonium, including mono-, di-, and trialkylammonium species, pyridinium, imidazolinium, amidinium, poly(ethyleneiminium), ammonioalkylsulfonate, ammonioalkylcarboxylate, amphoacetate, and poly(ethyleneoxy)sulfonyl moieties, are so excluded.

Under certain conditions as set forth below, amphiphilic compounds that otherwise meet the requirements of "oil" as set forth above may be considered "oils," and therefore are considered to be part of the oil portion. As such, the amphiphilic compounds that otherwise meet the requirements of "oil" and are not so amphiphilic so as to render the oil portion to be able to emulsify water. For example, the oil portion including the amphiphilic compound may not be capable of forming a water-in-oil emulsion (stable for one week at 20°C) when 1 part by weight of water is added to 2 parts of the oil portion and agitated in a manner typically suitable for forming a water-in-oil emulsion.

Compounds suitable for use in or as the oil portion include various hydrocarbons (straight or branched chain alkanes or alkenes, ketone, diketone, primary or secondary alcohols, aldehydes, sterol esters, alkanoic acids, turpenes, monoesters), such as those having a carbon chain length ranging from C₆-C₃₈, such as C₆-C₁₈. The oils may include ester and/or ether functional groups.

Suitable non-limiting examples of oil that may be included in the oil portion are alkyl diesters. One suitable alkyl diester is diisopropyl adipate, available as CRODAMOL DA from Croda Inc. of Edison, NJ (CRODAMOL DA has an interfacial tension as measured using the Pendant Drop Interfacial Tension Test of 11.6). Another suitable class of oils include the reaction product of glycols and fatty acids. One suitable reaction product of glycols and fatty acids is PPG-2 myristyl ether propionate available as Crodamol PMP from Croda Inc. of Edison, NJ (CRODAMOL PMP has an interfacial tension as measured using the Pendant Drop Interfacial Tension Test of 16.8). Another suitable class of oils includes triglycerides. One suitable triglyceride is a mixture of caprylic/capric triglycerides, available as Miglyol 812 from Sasol Olefins & Surfactants of Houston, Texas. Miglyol 812 has an interfacial tension as measured using the Pendant Drop Interfacial Tension Test of 24.7. Another suitable class of oils includes alkyl esters. One such suitable alkyl ester is isopropyl palmitate, available as Propal NF from the Lubrizol Corporation of Wickliffe, Ohio. Another suitable class of oils includes dialkyl carbonates. One suitable dialkyl carbonate is a dicaprylyl carbonate, available as CETIOL CC from Cognis Corp. of Ambler, Pennsylvania. CETIOL CC has an interfacial tension as measured using the Pendant Drop Interfacial Tension Test of 31.9. Another suitable class of oils includes alkyl benzoate esters. One suitable alkyl benzoate ester C₁₂-C₁₈ alkyl benzoate, available as FINSOLV TN (CAS# 68411-27-8, specific gravity 0.915-0.935; refractive index at 20C 1.43-1.487) from Innospec Active Chemicals of Edison, New Jersey, or Tegosoft TN from Evonik Goldschmidt GmbH of Essen, Germany. Tegosoft TN has an interfacial tension as measured using the Pendant Drop Interfacial Tension Test of 32.5.

As mentioned above, certain oils that have interfacial tension with water outside the ranges noted above (e.g., greater than 9 and less than 32.5) may be included in the oil portion as long as a sufficient percentage of the oil portion is formed from oils within the noted ranges. However, it is not suitable for the oil portion to consist entirely of oils having interfacial tension with water of 9 or less, or to include such oils at levels effective to lower the interfacial tension of the aggregate oil portion to less than 9. Similarly, it is not suitable for the oil portion to consist entirely of oils having interfacial tension with water of 32.5 or more, or to include such oils at levels effective to raise the interfacial tension of the aggregate oil portion to greater than 32.5.

Examples of oils having an interfacial tension with water of 9 or less, include a blend of C₁₂-C₁₅ alkyl benzoate, dipropylene glycol dibenzoate, and PPG-15 stearyl ether benzoate, available as FINSOLV TPP from Innospec Active Chemicals. Examples of oils having an interfacial tension with water of 32.5 or more, include C₁₂-C₁₅ alkyl benzoate, dimethicone (interfacial tension, I.T. = 45, as reported in K. Hughes, Vadim F. Lvovich, J. Woo, B.Moran, A.Suares, M.Truong, "Novel methods for emollient characterization", Cosmetics and Toiletries Manufacture Worldwide, 2006, 19-24), ethyl methicone (I.T. = 46.3, as measured by the applicants), diethylhexylcyclohexane available as CETIOL S from Cognis (I.T. = 49.8), and mineral oil (I.T. = 50, again as reported in K. Hughes, Vadim F. Lvovich, J. Woo, B.Moran, A.Suares, M.Truong, "Novel methods for emollient characterization", Cosmetics and Toiletries Manufacture Worldwide, 2006, 19-24).

Other oils suitable for use in the composition are organic UV-filters that are liquid at ambient temperature (22°C) and pressure (1 atmosphere). Liquid, organic UV-filters that are useful in the present invention are cosmetically-acceptable compounds that absorb radiation in the UV range and are generally soluble in one or more organic hydrocarbon solvents. The organic, UV-filter absorbs radiation in some portion of the ultraviolet spectrum (290nm-400nm), and may have an extinction coefficient of at least about 1000 mol⁻¹ cm⁻¹, for example greater than 10,000 or 100,000 or 1,000,000 mol⁻¹ cm⁻¹, for at least one wavelength within the above-defined ultraviolet spectrum. The liquid, organic UV-filter may co-exist with the oil portion and the C₂-C₃ mono-alcohol in a single phase.

Examples of liquid, organic UV-filters include, without limitation, esters of cinnamonic acid, in particular 4-methoxycinnamonic acid-2-ethylhexylester, 4-methoxycinnamonicacid propylester, 4-methoxycinnamic acid isoamyl ester, 2-cyano-3,3-phenylcinnamonic acid-2-ethylhexyl ester (octocrylene); esters of salicylic acid, i.e., salicylic acid-2-ethylhexylester, salicylic acid-4-isopropylbenzyl ester, salicylic acid homomenthyl ester, 3,3,5-Trimethylcyclohexyl 2-hydroxybenzoate (homosalate), dimethicodiethyl benzal malonate, and Polysilicone-15.

As one skilled in the art will readily appreciate, the term "organic UV-filter", as used herein, does not include ultraviolet-screening particles ("UV-screening particles") typically used at least in part to scatter ultraviolet radiation. Examples of UV-screening particles include inorganic oxides, including titanium dioxide, zinc oxide, iron oxides, silicone oxides, or other metal (e.g., transition metal, such as crystalline transition metal) oxides. As one skilled in the art will also readily appreciate, the term "solid, organic UV-filter", as used herein, does not include particulate organic UV-filters (e.g., such as particulate Bisoctrizole, available as TINOSORB M from BASF) that exist as dispersions of the solid particles in the composition (typically white in appearance), as opposed to compositions of the present invention where the UV-filter is dissolved in the composition. Compositions of the present invention may be substantially free of one or both of UV-screening particles and particulate organic UV-filters that exist as dispersions in the composition. The compositions of the invention may be free of such UV-screening particles and particulate organic UV-filters.

The liquid, organic, UV-filter may be present in the composition, or concentrate, in a range from about 0% to about 40%, such as from about 5% to about 35%, such as from about 10% to about 35%, e.g., from about 15% to about 35% by weight.

The inventors have found that it is possible to form compositions that include dissolved, solid, organic, UV-filters compositions that resist whitening in the presence of water, and are substantially free of a mixture of a low-volatility, C₂ - C₈ liquid silicone and a branched fatty acid ester of a polyprotic carboxylic acid in a ratio of from about 3:1 to 4.25:1. The composition may be substantially free of one or both of the low-volatility, C₂ - C₈ liquid silicone and the branched fatty acid ester of a polyprotic carboxylic acid.

Low-volatility, C₂ - C₈ liquid silicones have "low-volatility" in that they have a flash point above about 105°C, such as above about 150°C, such as above about 200°C. Such compounds have at least one siloxane (Si-O-Si) linkage and are liquid at room temperature (melting point is below 25°C). These compounds are generally soluble in the C₂-C₃ mono-alcohol. Low-volatility, C₂ - C₈ liquid silicone includes a C₂-C₈ functional group. They may have siloxy repeat units with pendant alkyl groups, such as those that include one or multiple units of:

- [CH₃-SiO-CₙH₂ₙ-CH₃] -

where n is from 2-8, such as from 2-4. One example is the compound in which n=2, available as SILWAX D02 (INCI: ethyl methicone) from Siltech of Dacula, Georgia. Other examples are caprylyl methicone and phenyl trimethicone.

Branched fatty acid esters of a polyprotic carboxylic acid ("BFEPCA") are branched fatty acid esters of a polyprotic carboxylic acid that are generally liquid at room temperature (melting point is below 25°C), water-insoluble and are soluble in the C₂-C₃ mono-alcohol. The BFEPCA may be a reaction product of a polyprotic acid with a C₁₀- C₃₀ fatty acid, such as a C₁₂- C₂₂ fatty acid. The fatty acid may be branched. The polyprotic acid may be selected from the group consisting of citric acid, ascorbic acid, phosphoric acid and sulfuric acid. The polyprotic acid may be citric acid or ascorbic acid. The BFEPCA may have five or more ester groups per molecule. One example of a BFEPCA is an octyldodecyl citrate polyester, which is commercially available as COSMOSURF CE-100 from SurfaTechCorporation/Siltech Corporation of Dacula, Georgia.

The oil portion may be present in the composition, or concentrate, in a range from about 8% to about 90%, such as from about 20% to about 75%, such as from about 30% to about 65%, e.g., from about 40% to about 55% by weight.

Furthermore, the inventors have found that it is necessary that compositions of the present invention have a sufficient amount of oil portion relative to the amount of C₂ or C₃ mono-alcohol. The volume fraction of oil relative to the sum of oil plus the C₂ or C₃ mono-alcohol (Vₒᵢₗ) is calculated by dividing the volume percentage of the oil portion in the composition, or concentrate, by the sum of the volume of the oil portion and the volume of the C₂ or C₃ mono-alcohol.

Specifically, Vₒᵢₗ should be about 0.44 or greater. Vₒᵢₗ may be from 0.44 to about 0.9, such as from about 0.47 to about 0.89, such as from about 0.47 to about 0.85, such as from about 0.47 to about 0.80. When a film-forming polymer that is insoluble in the oil portion but is soluble in the C₂ or C₃ mono-alcohol is present in the composition, Vₒᵢₗ may be greater than 0.50 and less than about 0.80, such as from 0.53 to about 0.65.

### Other Ingredients

Compositions of the present invention may include a film forming polymer to enhance film formation and provide some water resistance. By "film-forming polymer," it is meant a polymer that, when dissolved in the composition, permits a continuous or semi-continuous film to be formed when the composition is spread onto, e.g., smooth glass, and the liquid vehicle is allowed to evaporate. As such, the polymer should dry on the glass in a manner in which it should be predominantly continuous over the area upon which it is spread, rather than forming a plurality of discrete, island-like structures. Generally, the films formed by applying compositions on the skin according to the invention described herein, are less than, on average, about 100 microns in thickness, such as less than about 50 microns. The film-forming polymer may be insoluble in the oil portion (*i.e.,* is oil-insoluble), but is soluble in the C₂ or C₃ mono-alcohol.

Suitable film-forming polymers include natural polymers such as polysaccharides or proteins and synthetic polymers such as polyesters, polyacrylics, polyurethanes, vinyl polymers, polysulfonates, polyureas, polyoxazolines, and the like. Specific examples of film-forming polymers include, for example, acrylic homopolymers or copolymers with hydrophobic groups, such as acrylate/ocylacrylamide copolymers, including DERMACRYL 79, available from Akzo Chemical of Bridgewater, New Jersey; dimethicone/acrylates dimethicone copolymer, available as X-22-8247D from Shin-Etsu of Japan; hydrogenated dimer dilinoleyl/dimethylcarbonate copolymer, available from Cognis Corporation of Ambler, Pennsylvania as COSMEDIA DC; copolymer of vinylpyrrolidone and a long-chain alpha-olefin, such as those commercially available from ISP Specialty Chemicals of Wayne, New Jersey as GANEX V220; vinylpyrrolidone/tricontanyl copolymers available as GANEX WP660 also from ISP; water-dispersible polyesters, including sulfopolyesters such those commercially available from Eastman Chemical as EASTMAN AQ 38S. The film-forming polymer may be water-insoluble, but rendered soluble upon exposure to alkalinity in order to facilitate removal from the skin upon washing with soap. The amount of film-forming polymer present in the concentrate may be from about 0.25% to about 15%, or from about 0.5% to about 10%, or from about 1% to about 5%.

The composition may include a wax. By wax, it is meant one or more hydrophobic compounds that have a melting point (or melting range) that is in the range from 30°C to 120°C, such as in the range from 45°C to 100°C. The wax component may include a wax compound having a melting point from about 75°C to 100°C.

Suitable waxes include any of various hydrocarbons (straight or branched chain alkanes or alkenes, ketone, diketone, primary or secondary alcohols, aldehydes, sterol esters, alkanoic acids, turpenes, monoesters), such as those having a carbon chain length ranging from C₁₂-C₃₈. Also suitable are diesters or other branched esters. The compound may be an ester of an alcohol (glycerol or other than glycerol) and a C₁₈ or greater fatty acid.

Non-limiting examples include any of various natural waxes including lotus wax (e.g., Nelumbo Nucifera Floral Wax available from Deveraux Specialties, Silmar, California); beeswax (e.g., White Beeswax SP-422P available from Strahl and Pitsch of West Babylon, New York), insect waxes, sperm whale oil, lanolin, vegetable waxes such as canauba wax, jojoba oil, candelilla wax; mineral waxes such as paraffin wax; and synthetic waxes such as cetyl palmitate, lauryl palmitate, cetostearyl stearate, and polyethylene wax (e.g., PERFORMALENE 400, having a molecular weight of 450 and a melting point of 84°C, available from New Phase Technologies of Sugar Land, Texas); and silicone waxes such as C₃₀₋₄₅ Alkyl Methicone and C₃₀₋₄₅ Olefin (e.g., Dow Coming AMS-C30, having a melting point of 70°C, available from Dow Coming of Midland, Michigan). The wax component may include a high melting point ester of glycerol such as glycerol monostearate. The amount of wax may be present in the composition from about 0.1% to about 5%, or from about 0.1% to about 2%, or from about 0.1% to about 1%.

Any of various other cosmetically-acceptable ingredients may be included in the composition in amounts so as to not counter the effects of the various other ingredients. For example, ingredients such as fragrances, dyes, preservatives, skin benefit agents, photostabilizers, anti-oxidants may be includes, in, for example total concentrations that are less than about 10%, such as less than about 5%, such as about 2% or less, e.g., less than about 1%.

Compositions of the present invention are generally provided in the form of a single phase solution of various ingredients in the C₂ or C₃ mono-alcohol. The single phase solution may be prepared using techniques known in the art, such as by charging a vessel with C₂ or C₃ mono-alcohol and sequentially mixing/dissolving the various ingredients therein. Compositions of the present invention may be included as a concentrate in a product that additionally includes a propellant. The optional propellant generally exists as a vapor in equilibrium with liquid propellant that is dissolved in or is miscible with the remainder of the composition. The optional propellant aids in spraying the composition onto the skin. One suitable propellant is isobutene. The propellant may have finite (non-zero) solubility in water. Suitable examples of such propellants are dimethyl ether (which has a water solubility of 71 g/liter at 20 °C) and methyl ethyl ether.

The aerosol propellant may comprise about 10% to about 60%, or from about 20% to about 40%, or from about 25% to about 40% of the total weight of the concentrate plus propellant. Rather than include a conventional aerosol propellant, the composition may be propelled without ejected propellant using a "bag on valve" system which utilizes air or nitrogen that is isolated from the remainder of the composition. In use, compositions of the present invention are applied to skin, such as wet skin, and optionally rubbed onto the skin, in order to provide a protective UV sun-screening composition thereon.

### EXAMPLES

The following non-limiting examples further illustrate the claimed invention:

### Example I: Interfacial Tension Measurements and Ethanol Miscibility

Interfacial tension with water and ethanol miscibility were evaluated for a variety of cosmetic (emollient) oils as well as a mixture of liquid, organic UV-filters. Interfacial tension with water was evaluated using the Pendant Drop Interfacial Tension Test, as described above. Miscibility with ethanol was evaluated by visual examination of the oil ethanol mixtures. Oil/ethanol mixtures that separated into two phases after about one week exhibited a hazy or milky appearance when shaken due to the formation of an emulsion were considered to be immiscible. If the mixtures were clear, they were considered to be miscible. The results are reported in Table 1, below.

**Table 1: Interfacial Tension and Ethanol Miscibility of Oils**

| | Interfacial Tension, Mean | Interfacial Tension, Std Deviation | Miscibility with Ethanol |
|---|---|---|---|
| | (mN/m) | | |
| FINSOLV TPP | 9¹ | --- | Yes |
| CRODAMOL DA | 11.6 | 0.1 | Yes |
| PPG-2-Myristyl Ether Propionate | 16.8 | 0.1 | Yes |
| Caprylic/Capric Triglycerides | 24.7 | 0.2 | Yes |
| Isopropyl Palmitate | 28.6 | 0.1 | Yes |
| Cetiol CC | 31.9 | 0.3 | Yes |
| C₁₂-C₁₅ alkyl benzoate | 32.5 | 0.1 | Yes |
| CETIOL S | 49.8 | 0.5 | No |
| Oil blend, organic UV-filters and other oils² | 22.9 | 0.1 | Yes |

| | | | |
|---|---|---|---|
| ¹FINSOLV TPP was not measured for interfacial tension, but rather the data was taken from the supplier (Innospec) product literature. ²The oil blend, organic UV-filters and other oils, is a mixture that consisted of 3 liquid, organic UV-filters and 2 other oils - specifically; Homosalate;, Octisalate:, Octocrylene:, Butyloctyl Salicylate:, Corpan TQ, in a weight ratio of 15:5:10:5:0.1, respectively. | | | |

From the results, it can be noted that only the oil with the highest interfacial tension (Cetiol S) was not miscible with ethanol.

### Example II: Effect of Vₒᵢₗ and Interfacial Tension on Whitening

The effect of interfacial tension of various oils, as well as the volume fraction oil relative to oil plus ethanol (Vₒᵢₗ) were evaluated for effect on whitening in the presence of water. In order to facilitate sample preparation, two stock solutions were prepared:
Stock Solution 1: A solution of 3% avobenzone + 6% oxybenzone was prepared in ethanol and heated as needed to solubilize the solid, organic UV-filters.
Stock Solution 2: A solution of 3% avobenzone + 6% oxybenzone was prepared in each of the various oils to be tested and again heated as needed to solubilize the solid, organic UV-filters.

The stock solutions 1 and 2 were mixed in different volume proportions of Stock 1: Stock 2, to create mixtures with a calculated oil/oil+ethanol ratio (Vₒᵢₗ) values listed in the first column of Table 2, below. As such, all compositions included 3% avobenzone and 6% oxybenzone. Each composition included a mixture of ethanol with one oil, wherein the Vₒᵢₗ ranged from 0.091 to 0.91. Seven specific values for Vₒᵢₗ were tested for six different oils. Furthermore, to further assess the workable range, three of the six oils, were evaluated at the intermediate Vₒᵢₗ values of 0.43 and 0.47. Thus forty-eight (i.e., (7x6) + (2x3)) different compositions were evaluated.

Each of the mixtures was evaluated for whitening with a "Water Drop Whitening Test," whereby a 20 microliter drop of DI water is placed onto a smooth, black, flat, nonporous, hydrophobic surface that has been pre-cleaned with ethanol. A 20 microliter drop of the sample product is then added to the water droplet. The intensity of whiteness in the final drop is scored by visual inspection on a pass/fail scale, where pass (P) is clear like an oil drop, or very slightly hazy and fail (F) is whitening like a drop of skim milk at a dilution of up to 95% in water. The seventeen compositions with a score of P are Inventive examples (i.e., Ex. 1-Ex. 17). The thirty-one compositions with a score of F are Comparative examples (i.e., Comp. 1- Comp 31). The results are shown in Table 3, below:

**Table 2: Water Drop Whitening Test Results**

| **Vₒᵢₗ** | **Finsolv TPP** | **PPG-2 Myristyl Ether Propionate** | **Caprylyl Capric Triglycerides** | **Isopropyl palmitate** | **Dacaprylyl carbonate** | **C₁₂-C₁₈ Alkyl Benzoate** |
|---|---|---|---|---|---|---|
| | 9.0 | 16.8 | 24.7 | 28.6 | 31.9 | 32.5 |
| 1.0 | | | | | | |
| 0.91 | F | P | P | P | P | P |
| 0.80 | F | P | P | P | P | P |
| 0.50 | F | P | P | P | P | P |
| 0.47 | | P | | | P | F |
| 0.43 | | F | | | F | F |
| 0.40 | F | F | F | F | F | F |
| 0.33 | F | F | F | F | F | F |
| 0.20 | F | F | F | F | F | F |
| 0.091 | F | F | F | F | F | F |

### Example III: Low Polarity oils

To further assess the workable range of interfacial tension, four low polarity oils were evaluated. Comparative examples Comp. 32-35 were, respectively: dimethicone (interfacial tension of 45 as reported in the literature), ethyl methicone (interfacial tension of 46.3, as measured using Pendant Drop Interfacial Tension Test), diethylhexylcyclohexane available (CETIOL S from Cognis, interfacial tension of 49.8, as measured using Pendant Drop Interfacial Tension Test), and mineral oil (interfacial tension of 50 as reported in the literature). These low polarity oils could not sufficiently solubilize the 6% oxybenzone + 3% avobenzone, and/or were not miscible with ethanol. The Water Drop Whitening Test of Example II was attempted for mineral oil and dimethicone, but separation of oil and ethanol made proper evaluation impossible.

The results of Examples I and II reveal that in the samples that had no whitening, interfacial tension of the oil was greater than 9 and less than 32.5, and Vₒᵢₗ was greater than 0.44, and the oil portion was miscible with ethanol. Oils that were such low polarity so as to have interfacial tension of 45 or greater were not suitable for dissolving the solid sunscreen or for providing miscibility with ethanol.

### Example IV: Effect of Vₒᵢₗ and Interfacial Tension on Whitening For Compositions With Liquid, Organic UV-Filters

Additional compositions were evaluated for whitening. The compositions again included 6% oxybenzone and 3% avobenzone, but additionally included 24% liquid, organic UV-filters, 7% butyl octyl salicylate, 2% oil-insoluble film-forming polymer (DERMACRYL 79), 1% KP-545 (a film-forming polymer dissolved in cyclopentasiloxane), and diethyl hexyl 2,6, napthalate. As in the previous examples, Vₒᵢₗ was allowed to vary by adjusting the relative concentrations of ethanol to total oil. Specifically, ethanol concentration and isopropyl palmitate concentration were varied. Each of the mixtures was evaluated for whitening with the Pendant Water Drop Whitening Test. The formulations and whitening test results are shown in Table 3, below:

**Table 3: Formulations and Water Drop Whitening Test Results**

| **INCI Name** | **Ex. 18** | **Comp. 36** | **Comp. 37** | **Comp. 38** |
|---|---|---|---|---|
| SD Alcohol 40 B, 200 Proof (volatile solvent) | 48.3 | 56.3 | 53.3 | 51.3 |
| Acrylates/Octylacrylamide Copolymer (film-forming polymer) | - | - | - | - |
| Homosalate (organic UV-filter) | 15 | 15 | 15 | 15 |
| Oxybenzone (organic UV-filter) | 6 | 6 | 6 | 6 |
| Octisalate (organic UV-filter) | 5 | 5 | 5 | 5 |
| Avobenzone (organic UV-filter) | 3 | 3 | 3 | 3 |
| Octocrylene (organic UV-filter) | 4 | 4 | 4 | 4 |
| Diethylhexl 2,6 Naphtalate | 0.7 | 0.7 | 0.7 | 0.7 |
| Butyloctyl Salicylate | 7 | 7 | 7 | 7 |
| Cyclomethicone (and) Acrylates/ Dimethicone Copolymer (film-forming polymer) | 1 | 1 | 1 | 1 |
| Isopropyl palmitate | 10 | 2 | 5 | 7 |
| TOTAL | 100 | 100 | 100 | 100 |
| TOTAL OIL PORTION | 42.7 | 34.7 | 37.7 | 39.7 |
| OIL Portion + Ethanol | 91 | 91 | 91 | 91 |
| Vₒᵢₗ | 0.469 | 0.381 | 0.414 | 0.436 |
| Whitening Score | P | F | F | F |

It can be seen from the above results that, as isopropyl palmitate is added (increasing Vₒᵢₗ), the product becomes non-whitening. For compositions without oil-insoluble film-forming polymer, Vₒᵢₗ of 0.436 (Comp. 38) did not pass the whitening test, whereas Vₒᵢₗ of 0.469 (Ex. 18) did. This is consistent with Examples II and III and indicates that even though additional ingredients were introduced (liquid, organic UV-filters), the working ranges for Vₒᵢₗ are unchanged. The interfacial tension of the oil portion was believed to be in the range of 15 to about 30 for all test compositions.

In a related series of experiments, an oil-insoluble film-forming polymer (DERMACRYL) was incorporated into the compositions, which were then evaluated for whitening. The compositions and whitening test results are shown below in Table 4.

**Table 4: Formulations and Water Drop Whitening Test Results**

| **INCI/TRADE Name** | **Comp. 39** | **Comp. 40** | **Comp. 41** | **Comp. 42** | **Comp. 43** | **Ex. 19** | **Ex. 20** |
|---|---|---|---|---|---|---|---|
| SD Alcohol 40 B, 200 Proof Pharmco-Aaper (Shelbyville, KY) | 56.3 | 54.3 | 51.3 | 49.3 | 46.3 | 41.3 | 36.3 |
| Acrylates/Octylacrylamide Copolymer (oil-insoluble film-forming polymer); Dermacryl 79/Akzo (Bridgewater, NJ) | 2 | 2 | 2 | 2 | 2 | 2 | 2 |
| Homosalate (organic UV-filter); Eusolex HMS/Merck KGaA (Darmstdat, Germany) | 15 | 15 | 15 | 15 | 15 | 15 | 15 |
| Oxybenzone (organic UV-filter); Benzophenone -3/Uvinul M40 BASF (Florham Park, NJ) | 6 | 6 | 6 | 6 | 6 | 6 | 6 |
| Octisalate (organic UV-filter); Octyl Salicylate | 5 | 5 | 5 | 5 | 5 | 5 | 5 |
| Avobenzone (organic UV-filter); Neo heliopan 357/Symrise (Teterboro, NJ) | 3 | 3 | 3 | 3 | 3 | 3 | 3 |
| Octocrylene (organic UV-filter); Neo heliopan 303/Symrise | 4 | 4 | 4 | 4 | 4 | 4 | 4 |
| Corapan TQ/ Symrise | 0.7 | 0.7 | 0.7 | 0.7 | 0.7 | 0.7 | 0.7 |
| Halbrite BHB/Hallstar (Chicago, IL) | 7 | 7 | 7 | 7 | 7 | 7 | 7 |
| Cyclomethicone (and) Acrylates/ Dimethicone Copolymer (film-forming polymer); KP-545 / ShinEtsu (Tokyo, Japan) | 1 | 1 | 1 | 1 | 1 | 1 | 1 |
| Isopropyl palmitate | 0 | 2 | 5 | 7 | 10 | 15 | 20 |
| TOTAL | 100 | 100 | 100 | 100 | 100 | 100 | 100 |
| TOTAL OIL PORTION | 32.7 | 34.7 | 37.7 | 39.7 | 42.7 | 47.7 | 52.7 |
| OIL Portion + Ethanol | 89 | 89 | 89 | 89 | 89 | 89 | 89 |
| Vₒᵢₗ | 0.367 | 0.390 | 0.424 | 0.446 | 0.480 | 0.536 | 0.592 |
| Whitening Score | F | F | F | F | F | P | P |

It can also be seen that when the oil-insoluble, film-forming polymer was included, Vₒᵢₗ of 0.480 (Comp. 43) did not pass the whitening test, whereas Vₒᵢₗ of 0.536 (Ex. 19) did. As such, by adding an oil-insoluble, film-forming polymer, the working range is narrowed slightly.

## Claims

1. A composition comprising:
at least 10% by weight of a C₂ or C₃ mono-alcohol;
a solid organic UV-filter dissolved therein;
an oil portion comprising one or more oils, wherein the oil portion is miscible with the C₂ or C₃ mono-alcohol, wherein the oil portion has an interfacial tension with water of greater than 9 and less than 32.5, and
wherein the composition has a volume fraction of the oil portion relative to the oil portion plus the C₂ or C₃ mono-alcohol that is about 0.44 or greater, and wherein the composition is substantially free of a mixture of a low-volatility, C₂ - C₈ liquid silicone and a branched fatty acid ester of a polyprotic carboxylic acid in a ratio of from about 3:1 to 4.25:1, and wherein the composition is substantially anhydrous.

2. The composition of claim 1, wherein the composition is free of one or both of the low-volatility, C₂ - C₈ liquid silicone and the branched fatty acid ester of a polyprotic carboxylic acid.

3. The composition of claim 1 or claim 2, wherein the composition comprises 10% to about 56% of the C₂ or C₃ mono-alcohol, preferably 15% to about 56% of the C₂ or C₃ mono-alcohol, preferably 20% to about 56% of the C₂ or C₃ mono-alcohol.

4. The composition of any preceding claim, wherein the oil portion has an interfacial tension with water of from about 11.6 to about 32.5.

5. The composition of any preceding claim, wherein the oil portion has an interfacial tension with water that is equal to or greater than that of diisopropyl adipate and equal to or less than that of C₁₂-C₁₈ alkyl benzoate.

6. The composition of claim 1, wherein the oil portion has an interfacial tension with water of from about 11.6 to about 31.9, preferably from about 11.6 to about 25, preferably from about 11.6 to about 20.

7. The composition of any preceding claim, wherein the volume fraction of the oil portion relative to the oil portion plus the C₂ or C₃ mono-alcohol is from about 0.44 to about 0.9, preferably from about 0.47 to about 0.80.

8. The composition of any one of claims 1 to 6, wherein the volume fraction of oil relative to oil plus C₂ or C₃ mono-alcohol is from greater than 0.50 and less than about 0.80, preferably from about 0.53 to about 0.65.

9. The composition of claim 8, wherein the composition comprises an oil-insoluble, film-forming polymer.

10. The composition of any preceding claim, wherein the solid organic UV-filter is selected from the group consisting of avobenzone and oxybenzone.

11. The composition of any preceding claim, wherein the composition comprises from about 0.5% to about 20% of the solid, organic UV-filter dissolved therein.

12. The composition of any preceding claim, wherein the composition comprises from about 0.5% to about 9% of the solid, organic UV-filter dissolved therein.

13. The composition of any preceding claim, wherein the oil portion comprises a liquid, organic UV-filter.
